Europäisches Patentamt

European Patent Office

Office européen des brevets

(19)

(11) Publication number: **0 305 080**
**A2**

# EUROPEAN PATENT APPLICATION

(12)

(21) Application number: **88307387.6**

(22) Date of filing: **10.08.88**

(51) Int. Cl.⁴: **A61B 5/02**

(30) Priority: **23.08.87 IL 83615**

(43) Date of publication of application:
**01.03.89 Bulletin 89/09**

(84) Designated Contracting States:
**AT BE CH DE ES FR GB GR IT LI LU NL SE**

(71) Applicant: **Frank, Eyal**
**29 Hamered Street**
**Tel Aviv(IL)**

Applicant: **Reich, Yuval**
**Moshav Faran**
**Arava(IL)**

Applicant: **YISHUVEI POALEI AGUDAT ISRAEL**
**AGUDAH SHITUFIT MERCAZIT LTD**
**64 Frishman Street**
**Tel Aviv(IL)**

(72) Inventor: **Frank, Eyal**
**29 Hamered street**
**Tel Aviv(IL)**
Inventor: **Reich, Yuval**
**Moshav Faran**
**Arava(IL)**
Inventor: **Noy, Shraga**
**Kibbutz Hafetz Haim**
**Doar Hafetz Haim(IL)**

(74) Representative: **Morton, Colin David et al**
**Keith W Nash & Co. Pearl Assurance House**
**90-92 Regent Street**
**Cambridge CB2 1DP(GB)**

(54) **Portable apparatus for continuously monitoring peripheral blood flow.**

(57) Portable apparatus for monitoring peripheral blood flow comprising, according to one embodiment, apparatus for sensing infrared emissions from a bloodstream, including signal generating apparatus for generating a signal of an intensity corresponding to the intensity of an infrared emission sensed by the infrared sensing apparatus. According to an alternative embodiment, the apparatus for monitoring comprises apparatus for directing infrared radiation towards the bloodstream of a user; apparatus for receiving infrared radiation reflected from the bloodstream of the user; and apparatus associated with the apparatus for directing and the apparatus for receiving for measuring the difference between the infrared radiation transmitted to the bloodstream and the infrared radiation reflected from the bloodstream, including apparatus for generating a signal of an intensity corresponding to the intensity of the difference. The apparatus for monitoring also comprises a power source, electronic control apparatus selectably connectable to the power source including apparatus for receiving a signal from the signal generating apparatus and alarm apparatus associated with the signal receiving apparatus for emitting an alarm output if no signal of at least a selected minimum intensity is received by the signal receiving apparatus from the signal generating apparatus within a selected period of time.

FIG 1

## FIELD OF THE INVENTION

The present invention relates generally to devices for monitoring a person's pulse and, in particular, to portable devices for continuous monitoring of a person's pulse rate and peripheral blood flow rate.

## BACKGROUND OF THE INVENTION

There are known a variety of portable devices that monitor activity of the heart and which may be used by an unskilled person, some of which give a warning signal if the pulse rate of a user is above or below preset limits. Among known devices are those sensing pulse activity by means of pressure sensors, typically mounted in wristwatch fashion, which include the devices disclosed in U.S. Patents Nos. 3,742,937, 3,838,684, 4,038,976, 4,086,916, 4,406,290, and 4,450,843.

There are also known a number of devices for sensing pulse activity by means of electrodes placed on the skin, also typically mounted in wristwatch fashion, which includes devices disclosed in U.S. Patents Nos. 4,120,294, 4,129,125 and 4,230,127.

Further disclosed, in U.S. Patent No. 4,305,401, to Reissmuller et al, is a digital watch having a face mounted pulse sensor unit which is coupled to infrared plethysmograph electronics within the watch. Like the devices disclosed in the aforementioned U.S. Patents, Reissmuller's device is portable, allowing a wearer to function normally; it is relatively simple to operate; and it gives a readout of the pulse rate, when required, in the form of a digital display.

Disadvantages of the devices disclosed in the above-listed patents will be appreciated from the following summary of some heart conditions:

Cardiac arrest, in which the heart ceases to function, generally causes death if no action, such as cardiopulmonary resuscitation, (CPR), is taken within five minutes of its occurring. This is due to cutting off of the blood supply to vital organs, including the brain, which, within a very short period, may suffer irreversible damage, even if a person undergoes resuscitation.

It has been observed, however, that of persons suffering clinical death as a result of cardiac arrest, of those who received CPR within the first minute after its occurring, more than 60% were able to recover without cerebral damage. It is therefore vital that a condition of cardiac arrest be identified within the first minute of its occurring.

A situation in which a person suffers a heart attack, for example, and, as a result, his heart ceases to function efficiently, can quickly deteriorate and lead to a condition of cardiogenic shock. A symptom of cardiogenic shock is low blood pressure, which results in insufficient blood flow to vital organs, including the brain. It will be appreciated that it is vital to identify this condition before irreversible cerebral damage is caused.

In a case of hypovolemic shock, the flow of blood to the extremities of the body, including the brain, is reduced, due to a substantial loss of body fluids. The loss of fluids also ensures that whatever limited blood flow there is has an increased acidity, which can cause serious damage to various body organs, including the brain. By use of a prior art simple pulse meter an observer cannot predict brain damage resulting from reduced blood flow thereto.

It will be appreciated, therefore, that, in order to be able to predict or identify a situation of danger, of which those outlined above are examples, it is necessary to be able to receive a continuous indication of peripheral blood flow in addition to pulse rate.

Systems for continuous monitoring of blood pressure and pulse rate are disclosed in U.S. Patent No. 3,598,110 to Edmark and in U.S. Patent No. 4,083,366 to Gombrich et al. While the system of Edmark uses electrocardiogram apparatus (ECG) for monitoring heart activity, the system of Gombrich et al may use ECG, strain gauges or a mechanical diaphragm for this purpose.

Among disadvantages of the systems of Edmark and Gombrich et al, is that, in addition to being relatively expensive, they are both intended as diagnostic aids for use solely by physicians and other trained personnel. Their use also requires the relative immobility of a person whose heart activity is being monitored, a sitting or lying posture generally being adopted.

Yet a further system for monitoring heart functions of a person is disclosed in U.S. Patent No. 3,972,320. This system includes a monitoring system for producing an alarm at a central station when a monitored condition deviates beyond a predetermined limit. The monitor unit is portable and may take the form of a wristwatch and employs sensors, such as ECG type or acoustical transducers.

Among disadvantages of this system is that the monitoring is of pulse rate only and in addition, that there is no local indication of functions being monitored, an alarm signal being received only at the monitoring station.

## SUMMARY OF THE INVENTION

It is an aim of the present invention to provide portable apparatus for monitoring, by an unskilled person, of the peripheral blood flow rate in a subject.

It is also an aim that, when the peripheral blood flow rate is observed to be lower than a minimum selected level, the apparatus provides a warning indication to an observer, thus informing the observer of an impending condition of danger to a subject, such as cardiogenic or hypovolemic shock, thus providing a period of time in which action may be taken to prevent irreversible damage occurring as a result of the condition.

There is thus provided, in accordance with a preferred embodiment of the invention, portable apparatus for monitoring peripheral blood flow comprising apparatus for sensing infrared emissions from a bloodstream, including signal generating apparatus for generating a signal of an intensity corresponding to the intensity of an infrared emission sensed by the infrared sensing apparatus; a power source; electronic control apparatus selectably connectable to the power source including apparatus for receiving a signal from the signal generating apparatus; and alarm apparatus associated with the signal receiving apparatus, for emitting an alarm output if no signal of at least a selected minimum intensity is received by the signal receiving apparatus from the signal generating apparatus within a selected period of time.

Additionally in accordance with an embodiment of the invention, the apparatus for sensing and the signal generating apparatus are included in each of one or more infrared sensors configured to be fastened to the skin of a user and the alarm apparatus is operative to emit the alarm output if a signal of at least the minimum intensity is not received from each of the one or more sensors.

According to an alternative embodiment of the invention, there is provided portable apparatus for continuously monitoring peripheral blood flow, comprising apparatus for directing infrared radiation towards the bloodstream of a user; apparatus for receiving infrared radiation reflected from the bloodstream of the user; apparatus associated with the apparatus for directing and the apparatus for receiving for measuring the difference between the infrared radiation transmitted to the bloodstream and the infrared radiation reflected from the bloodstream, including apparatus for generating a signal of an intensity corresponding to the intensity of the difference; a power source; electronic control apparatus selectably connectable to the power source including apparatus for receiving the signal from the signal generating apparatus; and alarm apparatus associated with the signal receiving apparatus for emitting an alarm signal if no signal of at least a selected minimum intensity is received by the signal receiving apparatus from the signal generating apparatus within a selected period of time.

Additionally in accordance with the alternative embodiment of the invention, the apparatus for transmitting, the apparatus for receiving, the apparatus for measuring and the signal generating apparatus are included in each of one or more infrared sensors configured to be fastened to the skin of a user and the alarm apparatus is operative to emit the alarm output if a signal of at least the minimum intensity is not received from each of the one or more sensors.

Further in accordance with an embodiment of the invention, the alarm apparatus comprises audible output apparatus and visual output apparatus.

Additionally in accordance with an embodiment of the invention, the control apparatus also comprises apparatus associated with the signal receiving apparatus for monitoring the pulse rate of the user, the apparatus also comprising first apparatus associated with the control apparatus for providing a graphic display of the peripheral blood flow rate, selector apparatus associated with the control apparatus for setting an upper peripheral blood flow rate parameter, selector apparatus associated with the control apparatus for setting a lower peripheral blood flow rate parameter, selector apparatus associated with the control apparatus for setting an upper pulse rate parameter, selector apparatus associated with the control apparatus for setting a lower pulse rate parameter, second display apparatus associated with the control apparatus for providing a visual display of the pulse rate of the user and apparatus for selecting any one of first, second and third modes, wherein in the first mode the apparatus is disconnected from the power source, in the second mode the first display apparatus and the second display apparatus are operative to provide respective displays of the rate of peripheral blood flow and of the pulse rate of the user and in the third mode the apparatus for monitoring peripheral blood flow is operative to monitor the rate of peripheral blood flow and the pulse rate of the user, the first and second display apparatus being non-operative during the third mode.

## BRIEF DESCRIPTION OF THE DRAWINGS

The present invention will be understood and appreciated more fully from the following detailed description, taken in conjunction with the drawings, in which:

Fig. 1 is a schematic representation of peripheral blood flow monitoring apparatus, designed and constructed in accordance with a preferred embodiment of the invention;

Fig. 2 is an enlarged view of the control panel shown in Fig. 1;

Fig. 3 shows a pair of sensors suitable for use with the apparatus shown in Fig. 1;

Fig. 4 is a block diagram representation of the apparatus of Fig. 1;

Fig. 5 is a diagram of control and processing electronic circuitry used in the apparatus of Fig. 1;

Fig. 6 is a schematic representation of peripheral blood flow monitoring apparatus, designed and constructed in accordance with an alternative embodiment of the invention;

Fig. 7 is an enlarged view of the control panel shown in Fig. 6;

Fig. 8 shows a sensor suitable for use with the apparatus of Fig. 6; and

Fig. 9 is a block diagram representation of the apparatus of Fig. 6.

## DETAILED DESCRIPTION OF A PREFERRED EMBODIMENT

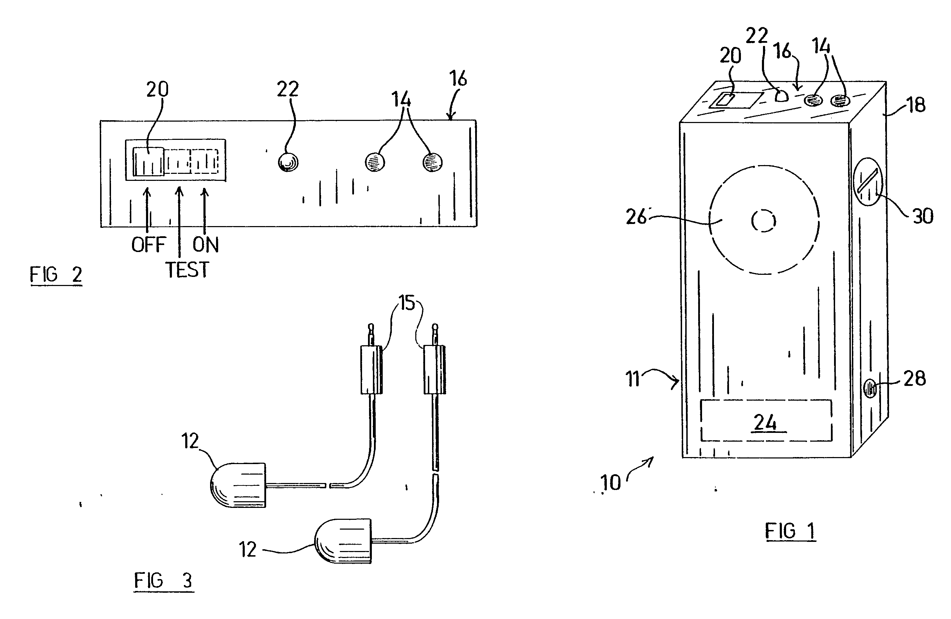

Referring to Figs. 1 to 3 there is shown monitoring apparatus 10 for monitoring the rate of peripheral blood flow of a user. Although the 'user' is taken to be a person, it is appreciated that this is in no way intended to limit applications of the monitoring apparatus of the present invention to human beings.

Apparatus 10 is intended for use by an unskilled person, and is designed so as not to limit the mobility or other functions of a user, while providing warning indications of impending conditions such as cardiogenic or hypovolemic shock.

Apparatus 10 is configured for use with a pair of infrared sensors (Fig. 3), referenced 12, that may be configured for attachment to any peripheral portion of the body. According to one embodiment of the invention, each of sensors 12 includes a light transmitting photodiode (not shown) which transmits either a continuous wave infrared signal or a pulsed infrared signal, while another photodiode, which is located adjacent the transmitting diode, receives the reflected infrared energy.

According to this embodiment, apparatus 10 indirectly measures, therefore, the peripheral blood flow by measuring the difference between the transmitted and reflected infrared radiation levels, the difference between the transmitted and reflected radiation levels corresponding to the rate of peripheral blood flow.

According to an alternative embodiment of the invention, each of sensors 12 directly measures the infrared emissions from the user's body, the measured levels of infrared emissions corresponding to the peripheral blood flow.

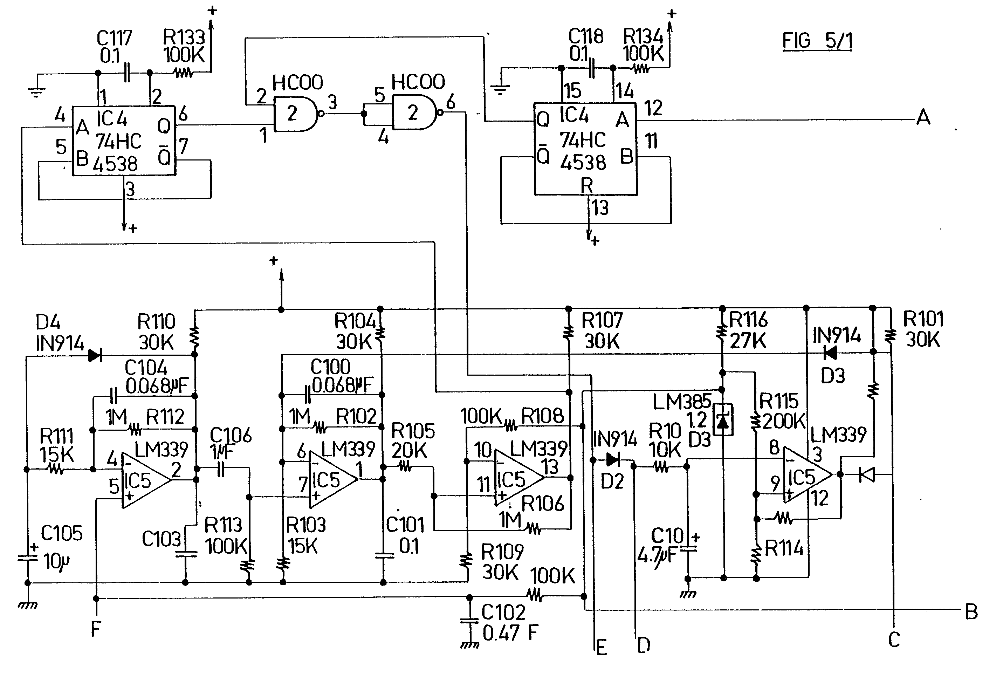

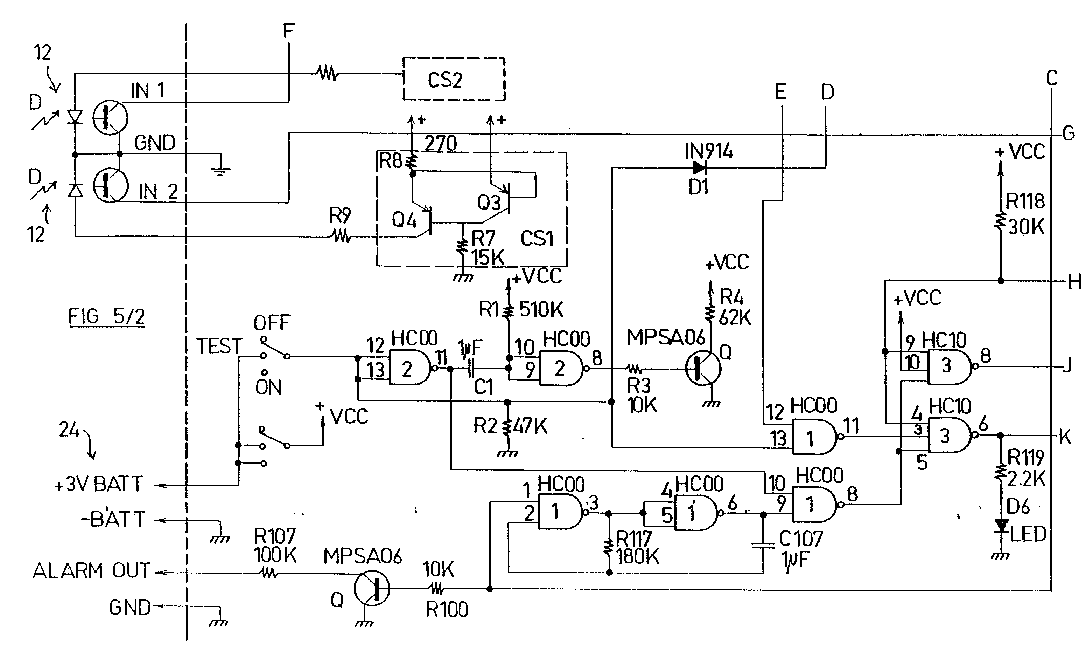

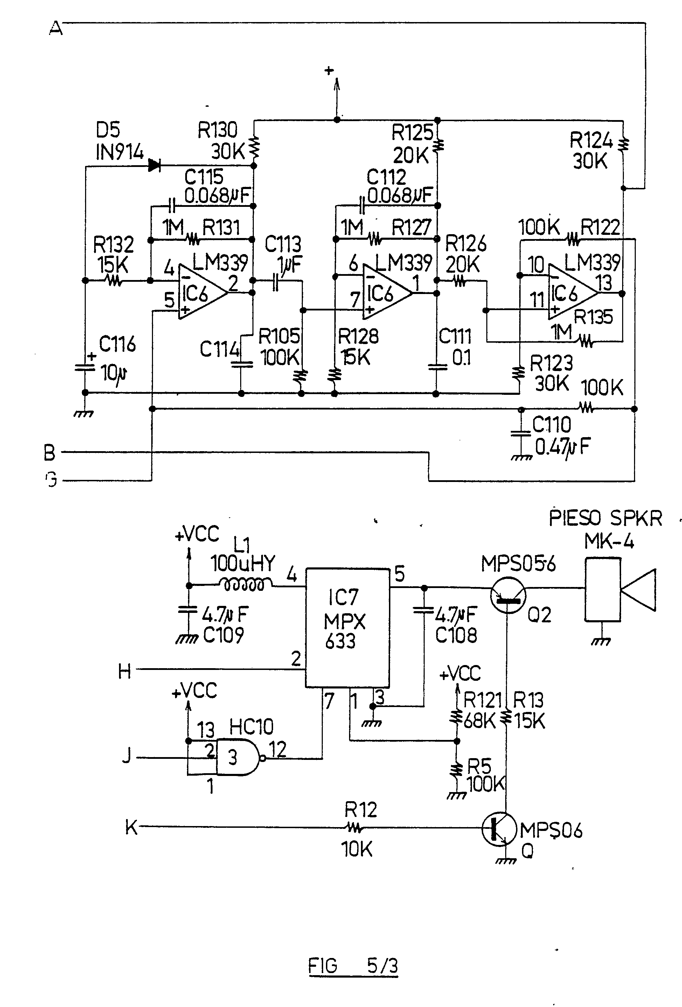

A control unit, referenced generally 11, includes a housing 18 for control and processing electronic circuitry, as schematically shown in Fig. 5. Mounted onto housing 18 is a control panel 16, in which are defined a pair of jacks 14 for receiving plugs 15 of sensors 12 and through which signals are transmitted from sensors 12 to control unit 11. Control panel 16 also includes a three-position switch 20 and an LED indicator 22.

Apparatus 10 is powered by a power supply 24, typically a pair of 1.5 V AA batteries, contained within housing 18. There is also provided a speaker 26, a jack 28, suitable for connection with external communications apparatus (not shown) and, in one embodiment of the invention, a sensitivity control switch 30, which governs the degree of sensitivity of sensors 12. In an alternative embodiment of the invention, the sensitivity level of sensors 12 is fixed and switch 30 is not provided.

In operation, sensors 12 are typically attached, such as by a glove like mounting defined thereby, to the fingers or toes of a user or to any other convenient body location, and control unit 11 may be attached, by means of a clip, for example, to the user's clothing.

Switch 20 has three positions, namely, an OFF position, a TEST position, and an ON position. In the OFF position apparatus 10 is switched off. In the TEST position, the batteries are checked by means of a checking circuit, forming part of the control and processing circuitry, as shown in Fig. 5, for a period of about 8 seconds. In the event that the batteries are not working properly, a continuous pitch is sounded by means of speaker 26. During the test period, the apparatus also provides visual and audible indications, by means of LED indicator 22 and speaker 26, respectively, of the heart beat of the user. This is facilitated by the measured arterial blood flow infrared emissions, each pulse of which corresponds to a heart beat.

After the apparatus has been fitted to the user and tested, the user's peripheral blood flow undergoes continuous monitoring when switch 20 is in the ON position. During this time, apparatus 10 does not emit any audible or visual indication, except in the event that the rate of peripheral blood flow falls below a certain preselected level, this being reflected by the level of infrared emissions generated thereby. In this case, a warning tone is emitted, this typically being a strong, broken pitch. In addition, LED indicator 22 flashes in accordance with the frequency of the pitch.

An important feature of the invention is that, although during normal operation of the apparatus no indications are given except in a case of a drop in the rate of peripheral blood flow below a selected rate, a warning is also given in the event that the apparatus becomes disconnected. This is due to receiving circuitry, as shown in Fig. 5, being operative to "expect" a signal of at least a minimum intensity, within a given period.

Thus, if, for any reason, including a break in contact between the sensors and the patient, or a break in contact between plugs 15 and jacks 14, no signal is received or a weak signal is received, an audible warning signal is emitted from speaker 26, as described above.

Although the apparatus is portable and does not require skilled personnel to operate it, it may be connected to a centralized monitoring station. This is facilitated by jack 28, and may be achieved by any suitable means, such as the transmission of a selected radio signal, simultaneous with the emission of warning indications locally, to a radio receiver which is connected to a telephone, which in turn may be connected to a computer forming part of a centralized monitoring station.

It should be noted that the term 'pulse' as employed below, unless stated otherwise, denotes the occurrence of a measured 'wave' of at least a minimum intensity of infrared emission, generated by pulsating blood circulation in the area of the sensors.

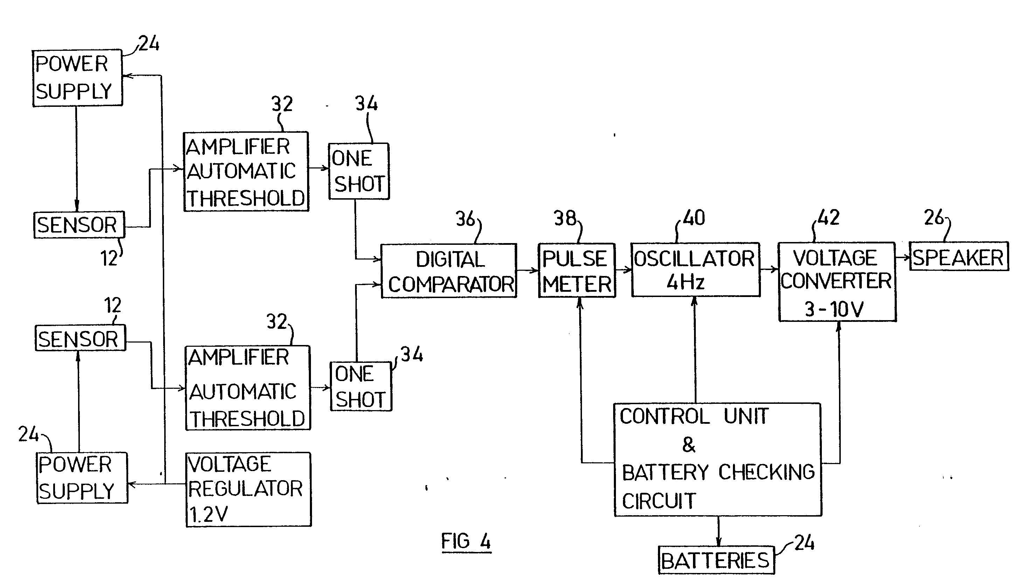

With additional reference to Figs. 4 and 5, the working of apparatus 10 will now be described. After apparatus 10 has been fitted to a user and switched on, each of sensors 12 transmits signals to an amplifier 32 having an automatic threshold.

In a situation of physical exertion by a user, a given pulse at the two sensor locations may occur at each sensor location at different times. One shot circuits, referenced 34, are provided, therefore, for lengthening the signal transmitted by sensors 12.

It is only once a given pulse of at least a minimum intensity is detected by both sensors that the two signals, which are first transmitted to a digital comparator 36, are then transmitted to a pulse meter 38 which measures the pulse rate. In the event that pulse meter 38 fails to detect a signal of at least a minimum intensity within a selected period after the previous detected signal, a warning procedure is effected.

The warning procedure is governed by an oscillator, referenced 40, which typically operates at a rate of 4 Hz and effects operation of a voltage converter 42. The voltage converter converts a battery voltage of about 3 V into a voltage of about 10 V, which is sufficient to generate a relatively strong audible alarm tone, by way of speaker 26.

Converter 42 also checks the voltage of the batteries over a continuous period. If the voltage of the batteries falls below 2 V a continuous tone is emitted from speaker 26, indicating that a change of battery is required.

In portable pulse monitoring apparatus of the prior art it is sufficient that there is a pulse, however weak, for an indication thereof to be displayed. In the present invention, however, the strength of the infrared emissions, generated by peripheral blood flow, is measured. In this way, if the intensity of the pulse is below a selected level, this being reflected in the intensity of the infrared emission, a condition of cardiogenic or hypovolemic shock can be detected sufficiently early in order to increase the possibility of complete recovery therefrom.

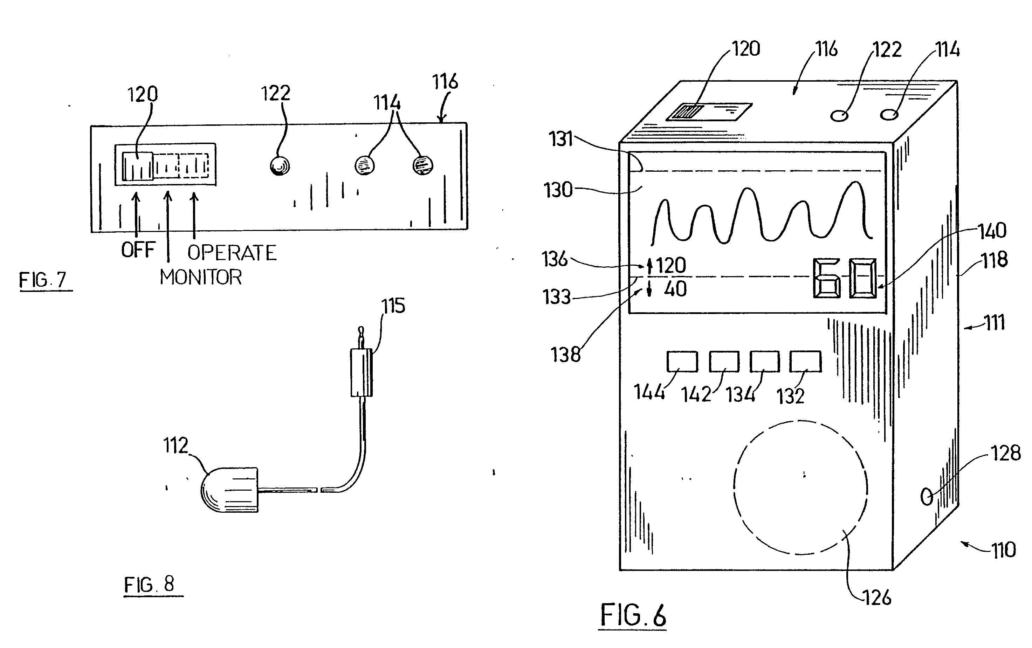

Reference is now made to Figs. 6 to 8, in which there is shown apparatus 110 for monitoring the peripheral blood flow of a user, constructed and operative in accordance with a further embodiment of the invention.

Apparatus 110 is configured for use with a single infrared sensor (Fig. 8), referenced 112, that may be configured for attachment to any peripheral portion of the body. Typically, it is configured for attachment to a finger. The sensor 112 is similar to each of sensors 12 as shown and described above in conjunction with Fig. 3 and its structure and function are not, therefore, specifically repeated herein.

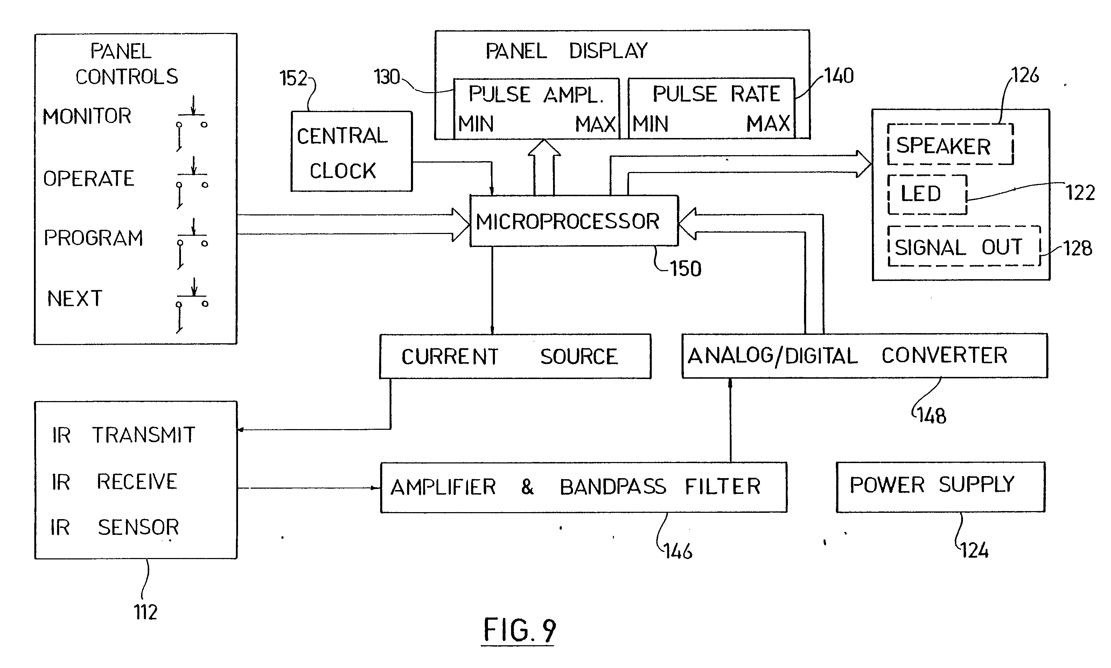

Apparatus 110 includes a control unit, referenced generally 111, which comprises a housing 118 for a microprocessor and associated apparatus, as shown schematically in Fig. 9. Mounted onto housing 118 is a control panel 116, in which there is provided a jack 114 for receiving plug 115 of sensor 112 and through which signals are passed from the sensor to control unit 111. Control unit 111 also includes a three-position operating switch 120 and an LED indicator 122, the respective functions of which are described below.

Apparatus 110 is powered typically by a pair of 1.5 V AA batteries (not shown) contained within housing 118. There is also provided a speaker 126 and a jack 128, suitable for connection with external communications apparatus (not shown).

Switch 120 has three positions, as stated, corresponding to three modes of operation of apparatus 110. The three positions are, as shown in Fig. 7, OFF, MONITOR and OPERATE. In the OFF position, apparatus 110 is nonoperational. In the MONITOR position, monitoring is carried out of the peripheral blood flow rate and of the pulse rate of a user. There is provided an LCD display 130 for providing a continuous graphical representation of the rate of peripheral blood flow. There are also provided selector buttons 132 and 134 for setting respective maximum and minimum peripheral blood flow limits which, in the shown embodiment, are displayed by respective lines 131 and 133 superimposed on display 130.

There are further provided selector buttons 142 and 144 for setting respective upper and lower pulse rate limits. These limits are displayed on display 130 as respectively indicated by reference numerals 136 and 138. In addition, the actual pulse rate of the user is also displayed, as indicated by reference numeral 140. According to one embodiment of the invention, indicator 122 may flash and an audible tone may be emitted via speaker 126 in accordance with the pulse rate of the user.

When switch 120 is in the OPERATE position, once the various limits have been set, although the peripheral blood flow rate and the pulse are being continuously monitored, no visual or audible display is provided by control unit 111. If, however, the peripheral blood flow rate or the pulse rate falls outside the respective preset limits, both visual and audible alarms signal are provided, respectively, by indicator 122 and speaker 126.

According to the present embodiment, from the commencement of an 'alarm' situation, as described, successively stronger audible signals continue to be emitted, for example, during the first and subsequent half minutes. If, after one or two minutes, no medical assistance has arrived, an alarm signal is emitted via jack 128 and suitable radio or telephone apparatus (not shown) connected therewith, to a central monitoring station.

With reference to Fig. 9, the working of apparatus 110 will now be described in further detail. After apparatus 110 has been fitted to a user and switched on, sensor 112 is operative to transmit infrared signals and to receive reflected infrared signals. The reflected infrared signals are passed to an amplifier and bandpass filter 146 which provides the amplified signals in analog form to an analog to digital converter 148. The signals are thereafter provided, in digital form, to a microprocessor 150.

The microprocessor, in association with which there is provided timing apparatus 152, is operative to evaluate the received data, calculating the pulse rate and the peripheral blood flow rate. As described above with reference to Fig. 6, when the apparatus is in a MONITOR mode of operation, the pulse rate and the peripheral blood flow rate are displayed on LCD display 130.

If the evaluated pulse rate or peripheral blood flow rate falls outside the preset parameters, alarm signals, as described above, are provided by way of speaker 126 or indicator 122. A signal to a remote monitoring station, as also described herein, may be provided via jack 128.

It will be appreciated by persons skilled in the art that the present invention is not limited by what has been particularly shown and described above. The scope of the invention is, rather, limited solely by the claims, which follow:

## Claims

1. Portable apparatus for monitoring peripheral blood flow comprising:
means for sensing infrared emissions from a bloodstream, including signal generating means for generating a signal of an intensity corresponding to the intensity of an infrared emission sensed by said infrared sensing means;
a power source;
electronic control means selectably connectable to said power source, including means for receiving a signal from said signal generating means; and
alarm means associated with said signal receiving means for emitting an alarm output if no signal of at least a selected minimum intensity is received by said signal receiving means from said signal generating means within a selected period of time.

2. Apparatus according to claim 1, and wherein said means for sensing and said signal generating means are included in at least one infrared sensor configured to be fastened to the skin of a user and said alarm means is operative to emit said alarm output if a signal of at least said minimum intensity is not received from each of said at least one sensor.

3. Apparatus according to either of claims 1 or 2, and also including means for governing the sensitivity of said means for sensing infrared emissions.

4. Portable apparatus for continuously monitoring peripheral blood flow, comprising:
means for directing infrared radiation towards the bloodstream of a user;
means for receiving infrared radiation reflected from the bloodstream of the user;
means associated with said means for directing and said means for receiving for measuring the difference between the infrared radiation transmitted to the bloodstream and the infrared radiation reflected from the bloodstream, including means for generating a signal of an intensity corresponding to the intensity of the difference;
a power source;
electronic control means selectably connectable to said power source, including means for receiving the signal from said signal generating means; and
alarm means associated with said signal receiving means for emitting an alarm signal if no signal of at least a selected minimum intensity is received by said signal receiving means from said signal generating means within a selected period of time.

5. Apparatus according to claim 4, and wherein said means for transmitting, said means for receiving, said means for measuring and said signal generating means are included in at least one infrared sensor configured to be fastened to the skin of a user and said alarm means is operative to emit said alarm output if a signal of at least said minimum intensity is not received from each of said at least one sensor.

6. Apparatus according to either of claims 4 or 5, and also including:
means for governing the level of infrared emissions transmitted by said means for transmitting and
means for governing the sensitivity of said means for receiving infrared radiation.

7. Apparatus according to any of the preceding claims, and wherein said alarm means comprises audible output means and visual output means and said electronic control means also includes pulse rate monitoring means operative to provide, via said respective audible and visual output means, audible and visual outputs corresponding to the pulse rate of the user.

8. Apparatus according to claim 7, and wherein said electronic control means also includes means associated with said alarm means for monitoring the power level of said power source, said alarm means being operative to provide an audible output when said power level is below a preselected level, said portable apparatus also comprising means for selecting any of first, second and third modes, wherein in said first mode said power supply is disconnected from said apparatus, in said second mode said electronic testing means and said pulse rate monitoring means are operative to respectively test the level of said power source and provide visual and audible outputs corresponding to the pulse rate of the user and in said third mode said apparatus for monitoring is operative to monitor the rate of peripheral blood flow of the user.

9. Apparatus according to any of the preceding claims, and also including external communications means for facilitating remote monitoring of the peripheral blood flow of a user.

10. Apparatus according to any of the preceding claims, and wherein said control means also comprises means associated with said signal receiving means for monitoring the pulse rate of the user, said apparatus also comprising:
first means associated with said control means for providing a graphic display of the peripheral blood flow rate,
selector means associated with said control means for setting an upper peripheral blood flow rate parameter,
selector means associated with said control means for setting a lower peripheral blood flow rate parameter,
selector means associated with said control means for setting an upper pulse rate parameter,
selector means associated with said control means for setting a lower pulse rate parameter,
second display means associated with said control means for providing a visual display of the pulse rate of the user and
means for selecting any one of first, second and third modes, wherein in said first mode said apparatus is disconnected from said power source, in said second mode said first display means and said second display means are operative to provide respective displays of the rate of peripheral blood flow and of the pulse rate of the user and in said third mode said apparatus for monitoring peripheral blood flow is operative to monitor the rate of peripheral blood flow and the pulse rate of the user, said first and second display means being nonoperative during said third mode.

FIG 1

FIG 2

OFF ON TEST

FIG 3

FIG 4

EP 0 305 080 A2

FIG 5/1

FIG 5/2

EP 0 305 080 A2

A

+

D5
IN914

R130
30K

R125
20K

R124
30K

C115
0.068µF

C112
0.068µF

1M  R131

R132
15K

C113
1µF

1M  R127

R126
20K

100K  R122

4  LM339
IC6  2

6  LM339
IC6  1

10  LM339
IC6  13

5  +

7  +

11  +

R135
1M

C116
10µ

C114

R105
100K

R128
15K

C111
0.1

R123
30K

100K

C110
0.47µF

B

G

PIESO SPKR
MK-4

+VCC  L1
100uHY  4

IC7
MPX
633

5

MPS056

Q2

4.7µF
C109

4.7µF
C108

H  2

+VCC

7  1  3

+VCC
R121  R13
68K  15K

13  HC10
2  3  12
1

R5
100K

K

R12

MPS06
Q

10K

FIG  5/3

FIG.6

FIG.7

OPERATE

MONITOR

OFF

FIG.8

FIG. 9